# EUROPEAN PATENT SPECIFICATION

(11) **EP 0 510 825 B1**
(45) Date of publication and mention of the grant of the patent: **03.07.1996**
(21) Application number: 92302917.7
(22) Date of filing: 02.04.1992
(51) Int. Cl.: B01J 29/40, C07C 209/60

(54) **Catalytic process for the production of ethyl amines**
Katalytische Herstellung von Äthylaminen
Procédé catalytique pour la production d' éthylamines

(30) Priority: 26.04.1991 ZA 913325
(43) Date of publication of application: 28.10.1992
(73) Proprietor: ZEOFUELS RESEARCH (PROPRIETARY) LIMITED, Krugersdorp, Transvaal (ZA)
(72) Inventor: Hutchings, Graham John, North Yorkshire DL6 3BA (GB); Themistocleous, Themistoclis, Sandton, Transvaal (ZA); Copperthwaite, Richard George, Johannesburg, Transvaal (ZA)
(74) Representative: Pearce, Anthony Richmond

(56) References cited:
- EP-A- 0 039 918
- EP-A- 0 077 016
- EP-A- 0 101 921
- EP-A- 0 305 564
- DE-A- 3 326 579
- JOURNAL OF THE CHEMICAL SOCIETY, CHEMICAL COMMUNICATIONS, 1987, Royal Societyof Chemistry MICHEL DEEBA et al. "Direct Amination of Ethylene by ZeoliteCatalysis"

## Description

This invention relates to a modified natural clinoptilolite for use as a catalyst in a process for the conversion of ethene and ammonia to ethyl amines, and to a method for the conversion of ethene and ammonia using the catalyst.

Ethyl amines are important chemical intermediates for use as starting materials for the manufacture of various solvents, pharmaceuticals, organic rubbers and surfactants. There is therefore a need to identify improved manufacturing processes for these chemicals. Traditional routes to the production on an industrial scale of lower alkyl amines usually involves the reaction of an alcohol with ammonia using an acidic catalyst. In many cases the alcohol is obtained by hydration of the corresponding alkene. There is therefore a need to identify a route to alkyl amines from the direct reaction of the alkene and ammonia. Early work on this route showed that alkali metal amide catalysts could be effective (see N B Gasc, A Lattes and J J Perie, Tetrahedron 39 (1983) 703-731; G P Pez US Patent No 4,302,603 ). While these catalysts provide reasonable performance there is a requirement to produce improved catalysts. In this respect the direct amination of alkenes using zeolite catalysts was identified by M Deeba, M E Ford and T A Johnson (J. Chem. Soc., Chem. Commun., (1987) 562-563), where it was reported that highest activities were recorded with medium pore size zeolites. The nature of the acidic sites and the shape selectivity imposed by the pore structure of the zeolite were considered to be important in limiting zeolite deactivation due to the competing alkene oligomerisation reaction. Subsequently, M Deeba and M E Ford extended their studies to demonstrate the efficacy of zeolite catalysts for the amination of isobutene (J Org. Chem. 53 (1988) 4594-4596), and they also showed that for the synthesis of ethyl amine the synthetic zeolite erionite in the hydrogen form gave particularly good life time and selectivity to the monoethylamine product. Subsequent to these studies W Holderich, M Hesse and F Naumann (Angewandte Chemie International Edition in English, 27 (1988) 226-246) and V Taglieber, W Holderich, R Kummer, W D Mross and G Saladia (German Patents Nos 3326579 and 3327000) have shown that alumino-, ferro- etc. and boro-substituted pentasil zeolites are also effective for alkene amination.

Under conditions normally employed in commercial catalysis the aforementioned zeolite catalysts tend to produce a mixture of alkyl amines. The chemical industry would prefer a catalyst that has high specificity to the desired product, i.e. the monoalkylamine, since this would reduce the product separation and manufacturing costs. Furthermore, the chemical industry would prefer to use a catalyst that is relatively inexpensive and this is not readily achieved if the aforementioned synthetic zeolites are utilized. In their initial disclosure, M Deeba, M E Ford and T A Johnson (J. Chem. Soc., Chem. Commun., (1987) 562-563) indicated that a natural zeolite clinoptilolite could also be used as a catalyst for this reaction. It is an object of this invention to provide a catalyst, based on a natural clinoptilolite, which has improved properties.

The modified natural clinoptilolite may be produced starting from any suitable natural clinoptilolite such as that from Zululand, South Africa, or that from Futatsui, Japan, by any suitable modification process. Various modification processes are set out below.

According to this invention there is provided a process for the conversion of ethene and ammonia to give a product containing at least 50% by weight, preferably at least 70% by weight, more preferably at least 90% by weight of monoethylamine in a reactor in the presence of a modified natural clinoptilolite which includes the steps of:
(a) feeding the ethene and the ammonia to the reactor containing the catalyst;
(b) converting the ethene and the ammonia in the reactor in the presence of the catalyst at a temperature of from 300°C to 600°C inclusive, preferably from 400°C to 500°C inclusive, and at a pressure from 101.3 to 10133 kPa (1 to 100 atmospheres) inclusive, preferably from 101.3 to 6078 kPa (1 to 60 atmospheres) inclusive; and
(c) recovering the product.

The invention is a process in which a modified natural clinoptilolite is used as a catalyst for the conversion of ethene and ammonia to ethyl amines where 50% or more by weight, preferably 70% or more by weight, more preferably 90% or more by weight of the ethyl amines is monoethylamine.

There are various processes by which the natural clinoptilolite may be modified to render it suitable for use as a catalyst, and these processes are set out below.

The first method is that disclosed in an article in Applied Catalysis, 16 (1985) 249-253, by Sakoh, Nitta and Aomura. This article discloses two methods for the modification of a natural clinoptilolite from Futatsui, Japan. The first method involves treating the natural clinoptilolite with 1M HCl at 80°C for 24 hours after which the sample is filtered off, washed with distilled water and dried in air. The second method involves impregnating the clinoptilolite with 0,05M and 0,5M H₂SO₄, whereafter the samples are filtered off, dried in air and then calcined at 400°C for 3 hours in air. These catalysts were utilised in the conversion of methanol to light olefins in a fixed bed continuous flow reactor under atmospheric pressure.

The second method is disclosed in South African Patent No 88/6733. This patent discloses a method for the modification of a natural clinoptilolite to produce a modified clinoptilolite for use in a reaction for the preparation of or transformation of hydrocarbons which method includes the step of treating the natural clinoptilolite with a suitable mineral acid such as hydrochloric acid at a concentration of greater than 1M, preferably from greater than 1M up to and including 2,5M, more preferably 2M, for a treatment time of longer than 24 hours, and at a suitable treatment temperature, preferably of from 40°C to 80°C, to produce the modified clinoptilolite. Further, after the acid treatment step, the clinoptilolite is preferably calcined at a suitable calcining temperature, e.g. from 450°C to 550°C, more preferably 500°C, for a suitable calcining time, e.g. 3 or 4 hours. The modified catalyst so produced may be used in a process for the conversion of methanol and/or dimethyl ether to hydrocarbon reaction products, and in a process for the cracking of hydrocarbon products.

The third method is disclosed in South African Patent No 89/3131. This patent discloses a method for the modification of a natural clinoptilolite to produce a modified clinoptilolite for use in a reaction for the preparation of hydrocarbons, which method includes the step of treating the natural clinoptilolite with a phosphorous containing acid such as phosphoric acid, pyrophosphoric acid, metaphosphoric acid, hypophosphorous acid, phosphorous acid or pyrophosphorous acid, at a concentration of 0,5M or greater, preferably from 0,5M up to and including 2M, for a treatment time of equal to or longer than 24 hours, preferably up to and including 96 hours, and at a suitable treatment temperature, preferably of from 40°C to 80°C inclusive, to produce the modified clinoptilolite. After the acid treatment step, the modified clinoptilolite may be calcined at a suitable calcining temperature of from 400°C to 550°C for a suitable calcining time from 3 hours, more preferably 4 hours. The modified clinoptilolite so produced may be used in a process for the conversion of methanol and/or dimethyl ether to hydrocarbon reaction products.

The fourth method is disclosed in South African Patent No 89/3132. This patent discloses a method for the modification of a natural zeolite to produce a modified zeolite for use in a reaction for the transformation of hydrocarbons which method includes the steps of treating the natural zeolite with a suitable alkali such as sodium hydroxide at a concentration greater than 0,5M preferably a concentration from 0,5M up to and including 5M, more preferably 2M, for a treatment time of longer than 1 hour preferably up to and including 48 hours, and at a suitable treatment temperature preferably from 30°C to 80°C inclusive, washing the resulting product, and treating the resulting product with a suitable mineral acid such as hydrochloric acid at a concentration of greater than 0,1M preferably a concentration from longer than 0,1 M up to and including 2M, for a treatment time of longer than 1 hour, preferably up to and including 48 hours, and at a suitable treatment temperature preferably from 40°C to 80°C inclusive, to produce the modified zeolite. Thereafter, the modified zeolite is preferably calcined at a suitable calcining temperature of from 400°C to 500°C for a suitable calcining time from 3 hours.

In terms of the present invention, the natural clinoptilolite may be modified either by (1) treating a natural clinoptilolite with a suitable mineral acid at a concentration of greater than 1M for a treatment time of longer than 24 hours and at a suitable treatment temperature or by (2) treating a natural clinoptilolite with a suitable alkali at a concentration greater than 0.5M for a treatment time of longer than 1 hour at a suitable temperature, washing the resulting product, and treating the resulting product with a suitable mineral acid at a concentration of greater than 0.1M for a treatment time of longer than 1 hour at a suitable temperature. The modified clinoptilolite may be produced starting from a natural clinoptilolite mined in Zululand, South Africa, or Fututsui, Japan, or from any other suitable natural clinoptilolite.

The invention is a process for the conversion of ethene and ammonia to give a product containing at least 50% by weight of monoethylamine in a reactor in the presence of a modified natural clinoptilolite which includes the steps of:
(a) feeding the ethene and the ammonia to the reactor containing the catalyst;
(b) converting the ethene and the ammonia in the reactor in the presence of the catalyst at a temperature of from 300°C to 600°C inclusive, preferably from 400°C to 500°C inclusive, and at a pressure of from 101.3 to 10133 kPa (1 to 100 atmospheres) inclusive, preferably from 101.3 to 6078 kPa (1 to 60 atmospheres) inclusive; and
(c) recovering the product.

The conversion of ethene and ammonia to ethyl amines is well known and may be carried out according to the method of the present invention using the known reaction conditions.

The reaction will generally be carried out in a fixed bed or a fluidized bed reactor at the temperatures and pressures mentioned above.

The crux of the use of the modified clinoptilolite of the invention is that high selectivity to the desired monoethylamine can be achieved at reasonable conversion of the ethene in the presence of an excess of ammonia together with minimal formation of other ethyl amines or hydrocarbon products.

Examples of the use of modified clinoptilolites as catalysts for the process of conversion of ethene and ammonia to ethyl amines will now be given.

### EXAMPLE 1

A sample (50g) of unmodified natural clinoptilolite, (denoted Catalyst 1), obtained from Zululand, South Africa, was suspended with stirring in a solution (500 ml) of 2M hydrochloric acid at 74°C for eight hours. Following this treatment the sample was collected by filtration and washed with de-ionised water. This treatment was repeated a further two times. The sample was washed well with de-ionised water, and dried at 120°C for four hours and then calcined at 500°C for four hours. Following this treatment, the modified clinoptilolite was denoted Catalyst 2 and was found to have a surface area of 85m²g⁻¹ by the BET method compared to the surface area of Catalyst 1 of 21m²g⁻¹. This acid treatment was found to increase the SiO₂/Al₂O₃ ratio of the zeolite without significant loss of crystallinity as measured by X-ray diffraction.

### EXAMPLE 2

A sample (50g) of unmodified clinoptilolite was suspended with stirring in an aqueous solution of sodium hydroxide (2M, 500 ml) at 50°C for eight hours. The sample was recovered by filtration and washed well with de-ionised water. The treated sample was suspended with stirring in an aqueous solution of ammonium sulphate (1M,1l) at 25°C for 40 minutes. The sample was recovered by filtration and washed well with de-ionised water, dried at 120°C for four hours and then calcined at 400°C for four hours. Following this treatment, the modified clinoptilolite was denoted Catalyst 3 and was found to have a surface area of 26m²g⁻¹. This treatment method was found to decrease the SiO₂/Al₂O₃ ratio of the zeolite without significant loss of crystallinity as measured by X-ray diffraction.

### EXAMPLE 3

A sample (50g) of unmodified clinoptilolite was suspended with stirring in an aqueous solution of phosphoric acid (H₂PO₄, 1M, 500 ml) at 60°C for forty-eight hours. Following this treatment the sample was recovered by filtration, washed well with de-ionised water, dried at 120°C for four hours and calcined at 400°C for five hours. This modified clinoptilolite was denoted Catalyst 4 and was found to have a surface area of 75m²g⁻¹. This treatment method was found significantly to increase the SiO₂/Al₂O₃ ratio of the zeolite without marked loss of crystallinity as measured by X-ray diffraction.

### EXAMPLE 4

A further sample of a modified clinoptilolite was prepared using a combination of acid and base treatments as follows. A sample (50g) of unmodified clinoptilolite was suspended with stirring an aqueous sodium hydroxide (2M, 500 ml) at 50°C for eight hours. The sample was collected by filtration and washed with de-ionised water and suspended with stirring in aqueous hydrochloric acid (0.5M 500 ml) for 15 hours at 60°C. The sample was collected by filtration, washed well with de-ionised water and dried at 120°C for four hours and then calcined at 400°C for five hours. The modified clinoptilolite so obtained was denoted Catalyst 5 and was found to have a similar SiO₂/Al₂O₃ ratio to that of the unmodified clinoptilolite and had a surface area of 60m²g⁻¹.

Catalysts 1 to 5 were tested for their effectiveness for the direct anination of ethene as follows using a fixed bed downflow microreactor. Ethene and ammonia were premixed (NH₃/C₂H₄ = 4:1 molar ratio) and were fed at a gas hourly space velocity of about 1000h⁻¹ measured at 20°C and 1 atmosphere pressure. The pressure in the catalytic reactor was maintained at 50 atmospheres total pressure using a back pressure regulator. Products were collected and analysed using standard gas chromatographic techniques. The results are set out in Table 1 below.

**TABLE 1**

| **Conversion of Ethene and Ammonia over Clinoptilolite Catalysts** | |
|---|---|
| CATALYST SAMPLE NO | CONVERSION OF ETHENE % |
| 1 | 7 |
| 2 | 15 |
| 3 | 17 |
| 4 | 18 |
| 5 | 18 |

The results shown in Table 1 indicate that the modified clinoptilolite catalysts are particularly effective for the direct amination of ethene as demonstrated by the increased conversion of ethene using Catalyst Samples Nos 2, 3, 4 and 5 as compared to the unmodified clinoptilolite Catalyst No 1.

For all modified clinoptilolite catalysts the selectivity to monoethylamine was found to be very high and specifically the ratio of monoethylamine to that of the diethylamine was greater than 20:1.

For comparative purposes a sample of the hydrogen form of an erionite zeolite was tested under comparable conditions to the catalysts Nos 1, 2, 3, 4 and 5 and an ethene conversion of about 14% was achieved with this material with similar selectivities to monoethylamine as obtained for catalysts 2, 3, 4 and 5. This demonstrates that the modified clinoptilotite catalysts of the invention give improved catalytic performance.

## Claims

1. A process for the conversion of ethene and ammonia to give a product containing at least 50% by weight of monoethylamine in a reactor in the presence of a modified natural clinoptilolite produced either by (1) treating a natural clinoptilolite with a suitable mineral acid at a concentration of greater than 1M for a treatment time of longer than 24 hours and at a suitable treatment temperature or by (2) treating a natural clinoptilolite with a suitable alkali at a concentration greater than 0,5M for a treatment time of longer than 1 hour and at a suitable treatment temperature, washing the resulting product, and treating the resulting product with a suitable mineral acid at a concentration of greater than 0,1M for a treatment time of longer than 1 hour and at a suitable treatment temperature, includes the steps of:
(a) feeding the ethene and ammonia to the reactor containing the catalyst;
(b) converting the ethene and the ammonia in the reactor in the presence of the catalyst at a temperature of from 300°C to 600°C inclusive, and at a pressure of from 101.3 to 10133 kPa (1 to 100 atmospheres) inclusive; and
(c) recovering the product.

2. A process according to claim 1 wherein the product contains 70% or more by weight of monoethylamine.

3. A process according to claim 1 wherein the product contains 90% or more by weight of monoethylamine.

4. A process according to claim 1 wherein the modified clinoptilolite is produced by treating the natural clinoptilolite with hydrochloric acid at a concentration of from greater than 1M up to and including 5M for a treatment time of longer than 24 hours and at a treatment temperature of from 40°C to 80°C inclusive.

5. A process according to claim 1 wherein the modified clinoptilolite is produced by treating a natural clinoptilolite with sodium hydroxide at a concentration of from greater than 0,5M up to and including 5M, for a treatment time of longer than 1 hour up to and including 48 hours and at a treatment temperature of 30°C to 80°C inclusive, washing the resulting product, and treating the resulting product with hydrochloric acid at a concentration of greater than 0,1M up to and including 2M for a treatment time of longer than 1 hour up to and including 48 hours at a treatment temperature of from 40°C to 80°C.

6. A process according to any one of claims 1 to 5 wherein the modified clinoptilolite is calcined at a suitable calcining temperature of from 400°C to 500°C inclusive for a suitable calcining time of from 3 hours.

7. A process according to any one of claims 1 to 6 wherein the modified natural clinoptilolite is produced starting from a natural clinoptilolite from Zululand, South Africa.

## Patentansprüche

1. Verfahren zur Umwandlung von Ethen und Ammoniak zu einem Produkt, welches mindestens 50 Gew.% Monoethylamin enthält in einem Reaktor in Gegenwart eines modifizierten natürlichen Clinoptilolits, welcher hergestellt wird entweder durch
(1) Behandeln eines natürlichen Clinoptilolits mit einer geeigneten Mineralsäure bei einer Konzentration von größer als 1M und einer Behandlungszeit von länger als 24 Stunden und bei einer geeigneten Behandlungstemperatur oder durch
(2) Behandeln eines natürlichen Clinoptilolits mit einem geeigneten Alkali bei einer Konzentration größer als 0,5M während einer Behandlungszeit von länger als 1 Stunde und bei einer geeigneten Behandlungstemperatur, Waschen des erhaltenen Produkts und Behandeln des erhaltenen Produkts mit einer geeigneten Mineralsäure bei einer Konzentration von größer als 0,1M während einer Behandlungszeit von länger als 1 Stunde und bei einer geeigneten Behandlungstemperatur,
welches die folgenden Stufen umfaßt:
(a) Einleiten des Ethens und des Ammoniaks in einen Reaktor, welcher den Katalysator enthält:
(b) Umwandeln des Ethens und des Ammoniaks in dem Reaktor in Gegenwart des Katalysators bei einer Temperatur von 300°C bis 600°C einschließlich und bei einem Druck von 101,3 bis 10133 KPa (1 bis 100 Atmosphären) einschließlich und
(c) Gewinnen des Produkts.

2. Verfahren gemäß Anspruch 1, wobei das Produkt 70 Gew.% oder mehr Monoethylamin enthält.

3. Verfahren gemäß Anspruch 1, wobei das Produkt 90 Gew.% oder mehr Monoethylamin enthält.

4. Verfahren gemäß Anspruch 1, wobei der modifizierte Clinoptilolit hergestellt wird durch Behandeln des natürlichen Clinoptilolits mit Salzsäure mit einer Konzentration von größer als 1M bis zu und einschließlich 5M während einer Behandlungszeit von länger als 24 Stunden und einer Behandlungstemperatur von 40°C bis 80°C einschließlich.

5. Verfahren gemäß Anspruch 1, wobei der modifizierte Clinoptilolit hergestellt wird durch Behandlung eines natürlichen Clinoptilolits mit Natriumhydroxid mit einer Konzentration von größer als 0,5M bis zu und einschließlich 5M, während einer Behandlungszeit von länger als einer Stunde bis zu und einschließlich 48 Stunden und bei einer Behandlungstemperatur von 30°C bis 80°C einschließlich, Waschen des erhaltenen Produkts und Behandeln des erhaltenen Produkts mit Salzsäure bei einer Konzentration von großer als 0,1M bis zu und einschließlich 2M während einer Behandlungszeit von länger als einer Stunde bis zu und einschließlich 48 Stunden bei einer Behandlungstemperatur von 40°C bis 80°C.

6. Verfahren gemäß einem der Ansprüche 1 bis 5, wobei der modifizierte Clinoptilolit bei einer geeigneten Calcinierungstemperatur von 400°C bis 500°C einschließlich während einer geeigneten Calcinierungszeit von über 3 Stunden calciniert wird.

7. Verfahren gemäß einem der Ansprüche 1 bis 6, wobei der modifizierte Clinoptilolit hergestellt wird ausgehend von natürlichem Clinoptilolit aus Zululand, Südafrika.

## Revendications

1. Procédé pour la conversion d'éthène et d'ammoniac pour donner un produit contenant au moins 50 % en poids de monoéthylamine, dans un réacteur, en présence d'une clinoptilolite naturelle modifiée produite soit (1) en traitant une clinoptilolite naturelle avec un acide minéral approprié à une concentration supérieure à 1 M pendant un temps de traitement supérieur à 24 heures et à une température de traitement appropriée, ou (2) en traitant une clinoptilolite naturelle avec un alcali approprié à une concentration supérieure à 0,5 M pendant un temps de traitement supérieur à 1 heure et à une température de traitement appropriée, en lavant le produit obtenu et en traitant le produit obtenu avec un acide minéral approprié à une concentration supérieure à 0,1 M pendant un temps de traitement supérieur à 1 heure et à une température de traitement appropriée, incluant les étapes consistant à :
(a) introduire l'éthène et l'ammoniac dans le réacteur contenant le catalyseur ;
(b) convertir l'éthène et l'ammoniac dans le réacteur en présence du catalyseur à une température de 300 °C à 600 °C inclusivement, et à une pression de 101,3 à 10133 kPa (1 à 100 atmosphères) inclusivement ; et
(c) récupérer le produit.

2. Procédé selon la revendication 1, dans lequel le produit contient 70 % en poids ou plus de monoéthylamine.

3. Procédé selon la revendication 1, dans lequel le produit contient 90 % en poids ou plus de monoéthylamine.

4. Procédé selon la revendication 1, dans lequel la clinoptilolite modifiée est produite en traitant de la clinoptilolite naturelle avec de l'acide chlorhydrique à une concentration supérieure à 1 M et jusqu'a 5 M y compris, pendant un temps de traitement supérieur à 24 heures et à une température de traitement de 40 °C à 80 °C inclusivement.

5. Procédé selon la revendication 1, dans lequel la clinoptilolite modifiée est produite en traitant une clinoptilolite naturelle avec de l'hydroxyde de sodium à une concentration supérieure à 0,5 M et jusqu'à 5 M y compris, pendant un temps de traitement supérieur à 1 heure et jusqu'à 48 heures y compris, et à une température de traitement de 30 °C a 80 °C inclusivement, en lavant le produit obtenu et en traitant le produit obtenu avec de l'acide chlorhydrique à une concentration supérieure à 0,1 M et jusqu'a 2 M y compris, pendant un temps de traitement supérieur à 1 heure et jusqu'à 48 heures y compris, à une température de traitement de 40 °C à 80 °C.

6. Procédé selon l'une quelconque des revendications 1 à 5, dans lequel la clinoptilolite modifiée est calcinée à une température de calcination appropriée, de 400 °C à 500 °C inclusivement, pendant un temps de calcination approprié allant à partir de 3 heures.

7. Procédé selon l'une quelconque des revendications 1 à 6, dans lequel la clinoptilolite naturelle modifiée est produite à partir d'une clinoptilolite naturelle de Zululand, Afrique du Sud.
